# DEMANDE DE BREVET EUROPEEN

(11) **EP 4 385 338 A1**
(43) Date de publication de la demande: **19.06.2024**
(21) Numéro de dépôt: 23217006.8
(22) Date de dépôt: 15.12.2023
(51) Int. Cl.: A23L 7/00, A23L 19/10

(54) **PROCÉDÉ DE PRODUCTION D'UN HYDROLYSAT**

(30) Priorité: 16.12.2022 BE 202206031
(71) Demandeur: Meurens Natural, 4650 Herve (BE)
(72) Inventeur: MALMENDIER, Yves Paul, 4910 Theux (BE)
(74) Mandataire: Calysta NV

(57) **Abrégé**

La présente invention se rapporte à un procédé de production d'un hydrolysat, à l'hydrolysat obtenu par le procédé selon l'invention et enfin à l'utilisation d'un tel hydrolysat pour la préparation d'un produit intermédiaire et/ou d'un produit final, par exemple un sirop, un sirop déshydraté/poudre, une base pour produits végétaux ou laitier, jus de fruit, jus de fruit concentré ou encore un produit alimentaire.

## Description

La présente invention se rapporte à un procédé de production d'un hydrolysat.

La présente invention concerne en outre un hydrolysat obtenu par le procédé selon l'invention.

Plus précisément, l'invention se rapporte à l'utilisation d'un tel hydrolysat pour la préparation d'un produit intermédiaire et/ou d'un produit final, par exemple un sirop, un sirop déshydraté/poudre, une base pour produits végétaux ou laitier, jus de fruit, jus de fruit concentré ou encore un produit alimentaire.

Les procédés d'hydrolyse sont bien connus de l'art antérieur, qu'ils soient à hydrolyse chimique ou encore à hydrolyse enzymatique.

Concernant les hydrolyses enzymatiques, il s'agit d'un procédé dans lequel des enzymes de type hydrolase, clivent un substrat en produits de réaction grâce à l'action d'une molécule d'eau. Les enzymes catalysent les réactions moléculaires, en les initiant, en accélérant leur déroulement et en s'assurant que le résultat est constant. En fin de réaction enzymatique, l'enzyme reste inchangée et peut donc catalyser une nouvelle réaction. Le substrat quant à lui peut être de différentes natures, par exemple protéique, saccharidique, lipidique, et de complexité variable.

Lorsqu'on s'intéresse par exemple au sirop de glucose, largement connu de l'industrie agroalimentaire et des glucosiers, la matière première telle que le blé, maïs ou céréales sont récoltées, broyées et tamisées afin de séparer l'amidon et protéines des autres composants des céréales, ce mélange amidon protéines est centrifugé avec de l'eau afin de séparer l'amidon des protéines. L'amidon récupéré est décomposé par hydrolyse enzymatique afin d'obtenir un hydrolysat qui servira ensuite à la fabrication de sirop de glucose.

De nos jours, les consommateurs ne sont plus nécessairement demandeur seulement de produits à base de sirop de glucose mais au contraire recherchent toujours plus de solutions alternatives.

Pour répondre à cette demande, l'industrie agroalimentaire a développé et continue de développer, à partir de matières premières variées, de nouvelles solutions de production d'hydrolysats, qui pourront par la suite être transformés pour obtenir les produits finaux répondant aux attentes des consommateurs.

De plus, l'industrie agroalimentaire est toujours à la recherche de nouvelles solutions innovantes pour la production d'hydrolysats ou de substrats, par exemple via le choix de matières premières variées, via l'amélioration de la qualité des hydrolysats et substrats fabriqués améliorant de fait la qualité des produits finis notamment des produits à haute teneur en sucres, à faible teneur en sucres, à haute valeur nutritionnelle, à haute teneur en fibres, en protéines.

Par exemple, dans le secteur agro-alimentaire, il est également d'intérêt croissant de pouvoir fournir des produits liquides ou solides aux consommateurs répondant à leurs attentes quant à la présence de sucres qu'ils soient ajoutés ou naturels. Ainsi, le secteur agroalimentaire est particulièrement demandeur de produits intermédiaires, d' hydrolysats qui serviront de base à la fabrication des produits finaux, répondant à ce besoin.

On comprend donc aisément que l'industrie agro-alimentaire doit faire de plus en plus face aux nouveaux challenges et exigences des consommateurs, en multipliant le nombre de procédés de production d'hydrolysats afin de pouvoir s'adapter aux différentes matières premières mais également aux propriétés recherchées.

En outre, l'industrie cosmétique est également demandeuse de nouveaux hydrolysats ou substrats afin de produire des produits de qualités répondant aux attentes toujours grandissantes des consommateurs. Dans ce cas, de manière similaire à l'industrie agroalimentaire, l'industrie cosmétique doit également faire face aux nouveaux challenges et exigences de consommateurs au vu des matières premières demandées pour la réalisation d'hydrolysat, ce qui entraînement une augmentation du nombre de procédés de production.

On connaît de l'état de la technique le document WO2017/165557 qui décrit un procédé de production d'hydrolysat à partir de composés de départ notamment de l'amidon et des fibres, une première enzyme, une deuxième enzyme et de l'eau, ces composés de départ sont introduits dans une unité dans laquelle a lieu l'hydrolyse.

On connaît également le document WO02/027011 qui divulgue un procédé de production d'hydrolysat dans lequel les farines et graines comme élément de départ sont lavées et traitées avec de l'éthanol dans un réservoir de mélange, des enzymes sont ensuite ajoutées.

En outre, le document WO2020/109541 décrit un procédé de préparation de fractions de céréales à haute valeur nutritionnelles qui comprend un broyage humine des grains en présence d'une composition enzymatique (malt ou dérivés du malt ou enzymes extraites du malt), tandis que le document WO2018/144621 divulgue quant à lui un procédé pour la production de liquides dérivés de substrats variés à haute teneur en fibres, protéines, faibles en carbohydrates. Ce document WO2018/144621 mentionne que le procédé utilise une hydratation contrôlée des graines ou légumes puis un broyage à sec ou humide pour exposer l'amidon et enfin l'ajout d'amylases pour convertir l'amidon en sucres.

Enfin, le document WO00/30457 quant à lui divulgue la préparation d'une suspension aqueuse d'avoine comprenant entre 5 et 45% de substrat d'avoine, puis le traitement de cette suspension par une enzyme.

On connaît également de l'art antérieur les documents suivants.

WO2009/045651 qui décrit un procédé de saccharification d'une biomasse utilisant un système de réacteur à hydrolyse alimenté et fonctionnant de façon discontinue, et divulguant qu'après quatre étapes d'ajouts discontinus de biomasse, le contenu du réacteur était majoritairement stagnant, l'essai s'étant poursuivi pendant 77 heures et la viscosité de l'hydrolysat étant devenue assez élevée ce qui a posé des problèmes de manipulation des fluides.

EP2582820 qui décrit un procédé d'hydrolyse enzymatique de cellulose dans lequel de la biomasse est ajoutée en continu dans un réacteur d'hydrolyse et la biomasse partiellement hydrolysée est en continu retirée du réacteur, pour alimenter de multiples réacteurs en série pour continuer l'hydrolyse.

US2022/015399 décrit quant à lui un procédé de formation d'une base d'avoine liquide concentrée à environ 12% de matière première dans l'eau.

Le document "Why on Earth Can People Need Continuous Recycle Membrane Reactors for Starch Hydrolysis ?" Paolucci-Jeanjean et al. divulgue un système d'alimentation à deux boucles de circulation dans lequel le substrat alimente une cuve enzymatique par un premier circuit de circulation, et la même cuve enzymatique est équipée d'un deuxième circuit de circulation, distinct du premier, permettant de faire une boucle avec l'installation équipée de membranes pour l'évacuation.

CN1330770 divulgue un procédé pour convertir de l'amidon granulaire en hydrolysat d'amidon soluble à une température inférieure à la température de gélatinisation initiale de l'amidon, dans lequel un mélange est fait d'une bouillie d'amidon avec plusieurs enzymes suivi d'un broyage et d'une ultrafiltration.

Bien que les procédés de production d'hydrolysats divulgués dans ces documents présentent certains avantages, ils gagneraient à être améliorés.

En effet, il s'avère que les procédés de production d'hydrolysat selon l'état de la technique manquent de polyvalence, et que bien souvent il est nécessaire d'adapter chaque procédé de production d'hydrolysat en fonction de chaque matière première.

En outre, on comprend qu'adapter chaque procédé en fonction de chaque matière première fait perdre en productivité et en rendement, d'autant plus lorsque l'on prend en considération l'augmentation constante des coûts des matières premières ainsi que des coûts de l'énergie dans de telles installations industrielles.

Il existe donc un réel besoin de fournir un procédé de production d'hydrolysat qui soit amélioré, polyvalent, qui puisse s'adapter à la large gamme de matières premières envisageables avec un contrôle de l'hydrolyse pour produire différents hydrolysats utilisables ultérieurement, et qui gagne en productivité et en rendement.

La présente invention a pour but de pallier les inconvénients de l'état de la technique en procurant un procédé de production d'un hydrolysat comprenant :
- une alimentation d'une unité de préparation de matière première,
- une alimentation d'une cuve à hydrolyse avec une phase aqueuse,
- une alimentation de la cuve à hydrolyse avec ladite matière première provenant de l'unité de préparation,
dans lequel,
- l'alimentation de la cuve à hydrolyse avec la matière première est réalisée par un enrichissement jusqu'à obtenir une concentration de matière première par rapport au poids d'eau compris entre 35% et 100%, de préférence compris entre 44% et 86%, comprenant :
   ∘ au moins un premier entraînement progressif et continu pendant une première période de temps prédéterminé, dans un circuit de circulation, de la matière première par la phase aqueuse pour former progressivement et en continu un premier mélange de matière première dans la phase aqueuse,
   ∘ au moins un deuxième entraînement progressif et continu pendant une deuxième période de temps prédéterminé, dans le circuit de circulation, de la matière première par le mélange de matière première dans la phase aqueuse et au moins une enzyme, pour former progressivement et en continu un deuxième mélange de matière première dans la phase aqueuse enzymatique,

dans lequel, ledit procédé comprend au moins une première étape d'ajout d'au moins une enzyme dans la cuve à hydrolyse qui a lieu entre ladite première période de temps prédéterminée et ladite deuxième période de temps prédéterminée,
et dans lequel l'hydrolysat est produit progressivement et en continu à partir du mélange de matière première dans la phase aqueuse enzymatique.

De préférence, le circuit de circulation du procédé selon la présente invention est une boucle de circulation entre la cuve à hydrolyse et l'unité de préparation.

Il est apparu de manière particulièrement avantageuse que fournir un procédé de production d'hydrolysat dans lequel une boucle de circulation est effectuée entre la cuve à hydrolyse et l'unité de préparation pour réaliser l'alimentation de la cuve à hydrolyse en matière première permettait d'enrichir progressivement et en continu la phase aqueuse en matière sèche.

En effet, le premier entraînement de la matière première par la phase aqueuse, dans la boucle de circulation entre la cuve à hydrolyse et l'unité de préparation de matière première, permet de former un premier mélange de matière première dans la phase aqueuse et de continuer à enrichir et concentrer progressivement et en continu ce mélange.

De manière particulièrement avantageuse, au moins une enzyme est ajoutée dans la cuve à hydrolyse après que le mélange de matière première dans la phase aqueuse ait commencé à se former. Ainsi, cela permet d'introduire l'au moins une enzyme au mélange de matière première dans la phase aqueuse au moment où ce mélange est encore faiblement concentré ou enrichi.

Ainsi, l'enrichissement de la phase aqueuse en matière première va se poursuivre en présence d'enzyme, dans la boucle de circulation entre la cuve à hydrolyse, le circuit de circulation et l'unité de préparation de matière première. De cette manière, le au moins un deuxième entraînement progressif et continu de la matière première dans la phase aqueuse enzymatique va permettre de former progressivement et en continu un deuxième mélange de matière première dans la phase aqueuse enzymatique.

Par conséquent, il est particulièrement avantageux que l'hydrolyse de la matière première débute dès l'introduction de ladite au moins une enzyme dans la cuve à hydrolyse, et va se poursuivre, de manière contrôlée lors de l'enrichissement. On comprend donc que l'hydrolysat est produit également de manière continue et progressive.

Le procédé selon la présente invention est particulièrement avantageux car il permet, d'une part d'enrichir progressivement et en continu la phase aqueuse en matière première, cela permettant d'éviter l'introduction brutale de la matière première dans son ensemble dans la cuve à hydrolyse comme cela est communément réalisé dans l'art antérieur, entraînant de laborieuses étapes de mélange, une augmentation brutale de la viscosité entraînant généralement une mauvaise circulation voire un bouchage des installations industrielles. D'autre part, en introduisant l'au moins une enzyme et en continuant l'étape d'enrichissement, on va progressivement et en continu continuer d'enrichir la phase aqueuse enzymatique en matière première en évitant les inconvénients mentionnés ci-avant, et enfin, l'hydrolyse de la matière première pour produire un hydrolysat va quant à elle également débuter et avoir lieu progressivement et en continu.

Cela permettant d'avoir un mélange de matière première dans la phase aqueuse enzymatique qui soit plus homogène, concentré et enrichi, par exemple jusqu'à obtenir une concentration d'au moins 25% de matière première dans la phase aqueuse enzymatique sans entraver le bon fonctionnement des appareils.

En outre, l'alimentation de la cuve à hydrolyse avec la matière première selon l'invention est réalisée par un enrichissement jusqu'à obtenir une concentration de matière première par rapport au poids d'eau compris entre 35% et 100%, de préférence compris entre 44% et 86%.

En outre, le procédé de production d'hydrolysat selon la présente invention en permettant de produire un mélange de matière première dans la phase aqueuse enzymatique qui soit plus homogène et plus concentré, permet également de produire un hydrolysat qui demandera moins d'eau à évaporer pour la préparation des produits intermédiaires ou finis ultérieurs et entraînant des économies non négligeables que ce soit au niveau de l'énergie nécessaire à l'évaporation ultérieure de l'eau de l'hydrolysat produit ou encore en terme de temps et de ressources nécessaires pour traiter les hydrolysats produits.

Enfin, le procédé selon la présente invention, en contrôlant le moment d'introduction de ladite au moins une enzyme dans la cuve à hydrolyse permet de contrôler l'hydrolyse de la matière première dans le temps, ce qui permet audit procédé selon l'invention d'être particulièrement adaptable quel que soit la matière première de départ, sans avoir à effectuer de modifications dans les installations industrielles.

Par conséquent, le procédé de préparation d'hydrolysat selon la présente invention est particulièrement avantageux en ce qu'il améliore la productivité et le rendement de la production d'hydrolysat, permettant de s'adapter à une large gamme de matières premières envisageables et de contrôler l'hydrolyse pour la production d' hydrolysats.

De préférence, le procédé selon la présente invention comprend au moins une deuxième étape d'ajout d'au moins une enzyme dans la cuve à hydrolyse, de préférence qui a lieu après la formation du deuxième mélange de matière première dans la phase aqueuse enzymatique.

L'ajout d'au moins une enzyme lors d'une deuxième étape d'ajout dans la cuve à hydrolyse, permet de manière avantageuse d'hydrolyser plus fortement le deuxième mélange de matière première dans la phase aqueuse enzymatique afin d'obtenir un hydrolysat selon la présente invention avec une valeur de Dextrose Equivalent (DE) plus haute, une valeur plus importante de sucres courts et donc in fine un hydrolysat avec un plus haut pouvoir sucrant.

Préférentiellement, l'au moins une enzyme ajoutée lors de la première étape d'ajout et/ou l'au moins une enzyme ajoutée lors de la deuxième étape d'ajout du procédé selon la présente invention, est ajoutée en quantité d'enzyme active comprise entre 0,002 et 0,1% en poids par rapport au poids de matière première.

Par exemple, l'au moins une enzyme ajoutée lors de la première étape d'ajout est ajoutée en quantité d'enzyme active comprise entre 0,001 et 0,05% en poids par rapport au poids de matière première.

Par exemple, l'au moins une enzyme ajoutée lors de la deuxième étape d'ajout est ajoutée en quantité d'enzyme active comprise entre 0,001 et 0,08% en poids par rapport au poids de matière première.

Avantageusement, l'au moins une enzyme du procédé selon la présente invention est choisie dans le groupe comprenant les hydrolases (3. -. -.-).

De manière avantageuse, l'au moins une enzyme du procédé selon la présente invention est choisie dans le groupe comprenant les estérases (3. 1. -.-), les glycosylases (3. 2. -.-), les éther-hydrolases (3. 3. -.-), les peptidases (3. 4. -.-), les enzymes de la classe 3. 5. -.-, les enzymes de la classe 3. 6. -.-, les enzymes de la classe 3. 7. -.-, les enzymes de la classe 3. 8. -.-, les enzymes de la classe 3. 9. -.-, les enzymes de la classe 3. 10. -.-, les enzymes de la classe 3. 11. -.-, les enzymes de la classe 3. 12. -.-, les enzymes de la classe 3. 13. -.-.

De préférence, la matière première du procédé selon la présente invention a subi un prétraitement de triage, nettoyage, épluchage, découpage, broyage, séchage, toastage, classification, traitements thermiques, floconnage, fractionnement, maltage et leurs mélanges. Préférentiellement, l'hydrolysat produit selon le procédé de la présente invention est un hydrolysat de tubercule comestible et/ou de graines d'une ou plusieurs plantes herbacées.

De préférence, de leur fraction et/ou de leur co-produit et/ou de leur sous-produit.

De manière préférée, l'hydrolysat de tubercule produit selon le procédé de l'invention est un hydrolysat de solanacées, d'apiacées, de convolvulaceae, d'astéracées, et leurs mélanges.

Avantageusement, l'hydrolysat de graines d'une ou plusieurs plantes herbacées du procédé selon l'invention est un hydrolysat de céréales, de pseudo-céréales, de poaceae, d'amaranthaceae, de polygonaceae, de fabaceae et leurs mélanges.

De manière particulièrement avantageuse, l'hydrolysat produit selon le procédé de l'invention est un hydrolysat de riz, d'avoine, de blé, de manioc, d'épeautre, de maïs, d'orge malté, de pomme de terre, de carotte, de panais, de patate douce, de topinambour, de poire de terre, de teff, de sorgho, de quinoa, de sarrasin, de seigle, de millet, d'orge, de yakon, de pois, de pois chiche, d'amaranthe, de blé dur, de tritordeum, de tournesol, et leurs mélanges.

On comprend du procédé de production d'hydrolysat selon la présente invention que ce dernier comprend une étape d'enrichissement comprenant au moins un premier entraînement progressif et continu pendant une première période de temps prédéterminé, et au moins un deuxième entraînement progressif et continu pendant une deuxième période de temps prédéterminé, ainsi qu'au moins une première étape d'ajout d'au moins une enzyme dans la cuve à hydrolyse qui a lieu entre la première période de temps prédéterminée et la deuxième période de temps prédéterminée.

Ainsi, prenant en compte la grande variété de matières premières pouvant être hydrolysées selon le procédé de l'invention, la première période de temps prédéterminé et la deuxième période de temps prédéterminé peuvent varier, dans le but de contrôler parfaitement l'hydrolyse de la matière première pour obtenir l'hydrolysat souhaité.

Il est par conséquent également envisagé, selon le procédé de la présente invention, que la deuxième période de temps prédéterminé soit nulle.

Dans un tel cas, le procédé selon la présente invention comprend :
- une alimentation d'une unité de préparation de matière première,
- une alimentation d'une cuve à hydrolyse avec une phase aqueuse,
- une alimentation de la cuve à hydrolyse avec ladite matière première provenant de l'unité de préparation,
dans lequel,
- l'alimentation de la cuve à hydrolyse avec la matière première est réalisée par un enrichissement jusqu'à obtenir une concentration de matière première par rapport au poids d'eau compris entre 35% et 100%, de préférence compris entre 44% et 86%, comprenant :
- au moins un premier entraînement progressif et continu pendant une première période de temps prédéterminé, dans un circuit de circulation, de la matière première par la phase aqueuse pour former progressivement et en continu un premier mélange de matière première dans la phase aqueuse,

dans lequel, ledit procédé comprend au moins une première étape d'ajout d'au moins une enzyme dans la cuve à hydrolyse qui a lieu après le début de la première période de temps prédéterminée,
et dans lequel l'hydrolysat est produit progressivement et en continu à partir du mélange de matière première dans la phase aqueuse enzymatique.

Ainsi, on comprend aisément que dans ce cas, l'au moins une enzyme peut être ajoutée lors de l'étape d'enrichissement, c'est-à-dire après que le premier entraînement progressif et continu de la matière première par la phase aqueuse ait commencé, afin que l'enrichissement de la phase aqueuse en matière première se poursuive en présence d'enzyme, dans la boucle de circulation entre la cuve à hydrolyse, le circuit de circulation et l'unité de préparation de matière première, permettant de concentrer le mélange et de produire l'hydrolysat de manière progressive et continue.

Il est également envisagé, lorsque le choix de matière première s'y prête, que l'au moins une enzyme est ajoutée après l'étape d'enrichissement, c'est-à-dire une fois que le mélange de la matière première dans la phase aqueuse dans la cuve à hydrolyse soit formé. En effet, il peut s'avérer que pour certaines matières premières, le mélange de matière sèche dans la phase aqueuse n'entrave pas le fonctionnement des installations industrielles par exemple à cause de la viscosité ou du bouchage des installations. Il est alors possible, préférentiellement que l'au moins une enzyme soit ajoutée directement au mélange dans la cuve à hydrolyse.

Cela étant particulièrement avantageuse pour la production d'hydrolysats nécessitant qu'un temps court d'hydrolyse.

Par exemple, l'au moins une enzyme ajoutée après l'étape d'enrichissement est ajoutée en quantité d'enzyme active comprise entre 0,002 et 0,1% en poids par rapport au poids de matière première.

De préférence, le procédé selon l'invention comprend au moins une première étape d'ajout d'au moins une enzyme dans la cuve à hydrolyse qui a lieu après la première période de temps prédéterminée ou entre la première période de temps prédéterminée et la deuxième période de temps prédéterminée.

D'autres formes de réalisation du procédé de production d'hydrolysat selon la présente invention sont indiquées dans les revendications annexées.

L'invention a aussi pour objet un hydrolysat obtenu par le procédé selon la présente invention.

De préférence, l'hydrolysat selon la présente invention est un hydrolysat de tubercule comestible et/ou de graines d'une ou plusieurs plantes herbacées.

Avantageusement, l'hydrolysat de tubercule produit selon la présente invention est un hydrolysat de solanacées, d'opiacées, de convolvulaceae, d'astéracées, et leurs mélanges.

Préférentiellement, l'hydrolysat de graines d'une ou plusieurs plantes herbacées selon l'invention est un hydrolysat de céréales, de pseudo-céréales, de poaceae, d'amaranthaceae, de polygonaceae, de fabaceae et leurs mélanges.

De manière avantageuse, l'hydrolysat selon l'invention est un hydrolysat de riz, d'avoine, de blé, de manioc, d'épeautre, de maïs, d'orge malté, de pomme de terre, de carotte, de panais, de patate douce, de topinambour, de poire de terre, de teff, de sorgho, de quinoa, de sarrasin, de seigle, de millet, d'orge, de yakon, de pois, de pois chiche, d'amaranthe, de blé dur, de tritordeum, de tournesol, et leurs mélanges.

D'autres formes de réalisation de l'hydrolysat selon la présente invention sont indiquées dans les revendications annexées.

L'invention a également pour objet une utilisation de l'hydrolysat selon la présente invention pour la préparation d'un sirop et/ou d'un sirop déshydraté et/ou d'une base pour produits végétaux.

De préférence, le sirop et/ou le sirop déshydraté et/ou la base pour produits végétaux de l'utilisation de l'hydrolysat selon l'invention, est utilisé dans la préparation d'un produit alimentaire choisi dans le groupe comprenant les boissons (notamment, soda, laitière, végétale) et/ou le jus de fruit et/ou le jus de fruit concentré, les desserts et yaourts (laitiers et végétaux), les biscuits, barres céréalières, en-cas, les céréales pour petit-déjeuner, les biscottes, crackers, les confiseries, les pains, pâtisseries, cakes, gaufres, les crèmes glacées, les sauces, les aliments pour bébé, les produits diététiques/sportifs, les préparations fruitées (notamment, confitures et compotes), et/ou dans la préparation d'un produit cosmétique.

### Description détaillée d'une réalisation de l'invention

D'autres caractéristiques et avantages de la présente invention seront tirés de la description non limitative qui suit, et en faisant référence aux exemples.

### Exemples

Une série d'hydrolysats ont été obtenus par le procédé selon la présente invention, par exemple un hydrolysat de tubercule comestible et/ou de graines d'une ou plusieurs plantes herbacées, plus particulièrement un hydrolysat à partir d'une farine de tubercule comestible et/ou d'une farine de graines d'une ou plusieurs plantes herbacées.

Plus particulièrement, la série d'hydrolysats a été obtenue à partir de solanacées, d'opiacées, de convolvulacées, d'astéracées, et leurs mélanges, de préférence à partir de leur farines respectives. Par exemple, l'hydrolysat est obtenu par le procédé de l'invention à partir de riz, d'avoine, de blé, de manioc, d'épeautre, de maïs, d'orge malté, de pomme de terre, de carotte, de panais, de patate douce, de topinambour, de poire de terre, de teff, de sorgho, de quinoa, de sarrasin, de seigle, de millet, d'orge, de yakon, de pois, de pois chiche, d'amaranthe, de blé dur, de tritordeum, de tournesol et leurs mélanges et/ou à partir de leur farine respective. De préférence, de leur fraction et/ou de leur co-produit et/ou de leur sous-produit.

Ainsi, la tubercule comestible et/ou les graines d'une ou plusieurs plantes herbacées, plus particulièrement sous forme de farine est introduite dans une unité de préparation de matière première. La cuve à hydrolyse quant à elle est alimentée avec une phase aqueuse.

L'unité de préparation de matière première est débouchant dans un circuit de circulation qui est une boucle de circulation entre l'unité de préparation et la cuve à hydrolyse. Ainsi, la phase aqueuse de la cuve à hydrolyse réalise une boucle de circulation entre la cuve à hydrolyse et le circuit de circulation ce qui permet l'entraînement progressif et continu de la matière première provenant de l'unité de préparation.

L'entraînement progressif et continu de la matière première dans la phase aqueuse permet de former un premier mélange de matière première dans la phase aqueuse, ce premier mélange qui va continuer à être enrichi et dont la concentration en matière première va progressivement et en continu augmenter au cours du temps, pendant une première période de temps prédéterminée.

Au moins une enzyme choisie dans le groupe des hydrolases est ajoutée à la cuve à hydrolyse et manière à former une phase aqueuse enzymatique. Ainsi, le mélange de matière première dans la phase aqueuse enzymatique effectue au moins un deuxième entraînement progressif et continu pendant une deuxième période de temps prédéterminée, afin de former un deuxième mélange de matière première dans la phase aqueuse enzymatique, permettant de former un hydrolysat de manière progressive et continue. Cela présentant l'avantage d'introduire l'au moins une enzyme au moment où le premier mélange est encore faiblement concentré et enrichi, de continuer à former progressivement, de concentrer et d'enrichir le mélange dans la phase aqueuse enzymatique et de débuter l'hydrolyse et la production d'hydrolysat qui est également produit de manière continue et progressive.

Cela permettant d'éviter l'introduction brutale de matière première dans la cuve à hydrolyse, ce qui entraîne généralement une augmentation brutale de la viscosité ce qui est indésirable pour les installations. Le procédé selon l'invention permet donc d'avoir une concentration en matière sèche dans la phase aqueuse d'au moins 25%, et une répartition homogène et uniforme.

En outre, la présente invention permet de contrôler l'hydrolyse et le profil de l'hydrolysat obtenu, et ce quel que soit la matière première utilisée et la qualité de l'hydrolysat recherchée, en fonction du moment d'introduction des enzymes, de la durée d'hydrolyse et du moment de l'arrêt de l'hydrolyse.

Il est également envisagé d'effectuer une deuxième étape d'ajout d'au moins une enzyme après la formation du deuxième mélange de matière première dans la phase aqueuse enzymatique, ce qui permet d'effectuer une deuxième hydrolyse par exemple pour obtenir un hydrolysat avec une valeur de Dextrose Equivalent plus importante, une teneur en sucres courts plus importante et in fine un hydrolysat obtenu selon l'invention avec un pouvoir sucrant plus important.

Ainsi, l'au moins une enzyme ajoutée lors de la première étape d'ajout est ajoutée à une teneur d'enzyme active comprise entre 0,001% et 0,05% en poids par rapport au poids de matière première. L'au moins une enzyme ajoutée lors de la deuxième étape d'ajout est ajoutée à une teneur d'enzyme active comprise entre 0,001% et 0,08% en poids par rapport au poids de matière première.

Avantageusement, la teneur totale de l'au moins une enzyme ajoutée pour l'hydrolyse du procédé selon l'invention est comprise entre 0,002 et 0,1% en poids d'enzyme active par rapport au poids de matière première, que ce soit pour la première étape d'ajout et/ou la deuxième étape d'ajout.

Le procédé selon la présente invention permet d'obtenir un hydrolysat, de manière contrôlée et stable au cours du temps, et ce quel que soit la matière première de départ, avec un unique procédé et au sein d'une seule installation industrielle, évitant ainsi de considérablement avoir à adapter l'installation industrielle en fonction de la matière première choisie comme cela est généralement le cas dans l'art antérieur.

Il s'avère qu'une hydrolyse en deux étapes, c'est-à-dire une première étape d'hydrolyse progressive et continue du deuxième mélange, et une deuxième étape d'hydrolyse dans la cuve à hydrolyse après la formation du deuxième mélange est particulièrement adapté à certaines matières premières.

En revanche, pour certaines autres matières premières, il peut être avantageux d'effectuer l'étape d'hydrolyse en une seule étape, pendant ou après que le mélange de matière première dans la phase aqueuse ait été formé de manière progressive et continue selon la présente invention.

Dans ce cas, la deuxième période de temps prédéterminée est nulle et l'étape d'ajout d'enzyme est effectuée pendant ou après que le mélange de matière première dans la phase aqueuse ait été effectué. Cela étant particulièrement avantageux pour la production d'hydrolysats à partir de matière première qui ne nécessitent qu'un temps court d'hydrolyse, afin de pouvoir contrôler l'hydrolyse de manière optimale et les qualités recherchés pour l'hydrolysat obtenu.

En outre, dans ce cas l'au moins une enzyme ajoutée pendant ou après la formation du mélange de matière première dans la phase aqueuse est ajoutée en quantité d'enzyme active comprise entre 0,002 et 0,1% en poids par rapport au poids de matière première.

Plus particulièrement, l'au moins une enzyme est choisie dans le groupe comprenant les hydrolases (3. -. -.-) et encore plus précisément dans le groupe comprenant les estérases (3. 1. -.-), les glycosylases (3. 2. - .-), les éther-hydrolases (3. 3. -.-), les peptidases (3. 4. -.-), les enzymes de la classe 3. 5. -.-, les enzymes de la classe 3. 6. -.-, les enzymes de la classe 3. 7. -.-, les enzymes de la classe 3. 8. -.-, les enzymes de la classe 3. 9. -.-, les enzymes de la classe 3. 10. -.-, les enzymes de la classe 3. 11. -.-, les enzymes de la classe 3. 12. -.-, les enzymes de la classe 3. 13. -.-.

En outre, la matière première de l'invention peut avantageusement subir un prétraitement tel qu'un triage, nettoyage, épluchage, découpage, broyage, séchage, toastage, classification, traitements thermiques, floconnage, fractionnement et leurs mélanges.

Ainsi l'hydrolysat obtenu par le procédé selon la présente invention est un hydrolysat pour lequel l'hydrolyse a été parfaitement contrôlée, et qui présente une valeur de Brix, de Dextrose Equivalent, un pouvoir sucrant, une viscosité, une couleur, une texture souhaité en vue de ses utilisations ultérieures.

En effet, l'hydrolysat obtenu selon la présente invention est particulièrement utilisé dans la préparation d'un sirop et/ou d'un sirop déshydraté et/ou d'une base pour produits végétaux.

Plus particulièrement, l'hydrolysat obtenu selon la présente invention est utilisé dans la préparation directe ou indirecte d'un produit alimentaire choisi dans le groupe comprenant les boissons (notamment, soda, laitière, végétale), et/ou le jus de fruit et/ou le jus de fruit concentré, les desserts et yaourts (laitiers et végétaux), les biscuits, barres céréalières, en-cas, les céréales pour petit-déjeuner, les biscottes, crackers, les confiseries, les pains, pâtisseries, cakes, gaufres, les crèmes glacées, les sauces, les aliments pour bébé, les produits diététiques/sportifs, les préparations fruitées (notamment, confitures et compotes), et/ou dans la préparation d'un produit cosmétique.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Procédé de production d'un hydrolysat comprenant :
- une alimentation d'une unité de préparation de matière première,
- une alimentation d'une cuve à hydrolyse avec une phase aqueuse,
- une alimentation de la cuve à hydrolyse avec ladite matière première provenant de l'unité de préparation,
dans lequel,
- l'alimentation de la cuve à hydrolyse avec la matière première est réalisée par un enrichissement jusqu'à obtenir une concentration de matière première par rapport au poids d'eau compris entre 35% et 100%, de préférence compris entre 44% et 86%, comprenant :
∘ au moins un premier entraînement progressif et continu pendant une première période de temps prédéterminé, dans un circuit de circulation, de la matière première par la phase aqueuse pour former progressivement et en continu un premier mélange de matière première dans la phase aqueuse,
∘ au moins un deuxième entraînement progressif et continu pendant une deuxième période de temps prédéterminé, dans le circuit de circulation, de la matière première par le mélange de matière première dans la phase aqueuse et au moins une enzyme, pour former progressivement et en continu un deuxième mélange de matière première dans la phase aqueuse enzymatique,
dans lequel, ledit procédé comprend au moins une première étape d'ajout d'au moins une enzyme dans la cuve à hydrolyse qui a lieu entre ladite première période de temps prédéterminée et ladite deuxième période de temps prédéterminée,
et dans lequel l'hydrolysat est produit progressivement et en continu à partir du mélange de matière première dans la phase aqueuse enzymatique.

2. Procédé selon la revendication 1, dans lequel ledit circuit de circulation est une boucle de circulation entre ladite cuve à hydrolyse et ladite unité de préparation.

3. Procédé selon la revendication 1 ou 2, lequel comprend au moins une deuxième étape d'ajout d'au moins une enzyme dans la cuve à hydrolyse, de préférence qui a lieu après la formation du deuxième mélange de matière première dans la phase aqueuse enzymatique.

4. Procédé selon l'une quelconque des revendications 1 à 3, dans lequel ladite au moins une enzyme ajoutée lors de la première étape d'ajout et/ou ladite au moins une enzyme ajoutée lors de la deuxième étape d'ajout, est ajoutée en quantité d'enzyme active comprise entre 0,002 et 0,1% en poids par rapport au poids de matière première.

5. Procédé selon l'une quelconque des revendications 1 à 4, dans lequel ladite au moins une enzyme est choisie dans le groupe comprenant les hydrolases (3. -. -.-).

6. Procédé selon la revendication 5, dans lequel ladite au moins une enzyme est choisie dans le groupe comprenant les estérases (3. 1. -.-), les glycosylases (3. 2. -.-), les éther-hydrolases (3. 3. -.-), les peptidases (3. 4. -.-), les enzymes de la classe 3. 5. -.-, les enzymes de la classe 3. 6. -.-, les enzymes de la classe 3. 7. -.-, les enzymes de la classe 3. 8. -.-, les enzymes de la classe 3. 9. -.-, les enzymes de la classe 3. 10. -.-, les enzymes de la classe 3. 11. -.-, les enzymes de la classe 3. 12. -.-, les enzymes de la classe 3. 13. -.-.

7. Procédé selon l'une quelconque des revendications 1 à 6, dans lequel la matière première a subi un prétraitement de triage, nettoyage, épluchage, découpage, broyage, séchage, toastage, classification, traitements thermiques, floconnage, fractionnement, maltage et leurs mélanges.

8. Procédé selon l'une quelconque des revendications 1 à 7, dans lequel l'hydrolysat produit est un hydrolysat de tubercule comestible et/ou de graines d'une ou plusieurs plantes herbacées, de préférence ledit hydrolysat de tubercule produit est un hydrolysat de solanacées, d'opiacées, de convolvulaceae, d'astéracées, et leurs mélanges et/ou ledit hydrolysat de graines d'une ou plusieurs plantes herbacées est un hydrolysat de céréales, de pseudo-céréales, de poaceae, d'amaranthaceae, de polygonaceae, de fabaceae et leurs mélanges.

9. Procédé selon la revendication 8, dans lequel ledit hydrolysat produit est un hydrolysat de riz, d'avoine, de blé, de manioc, d'épeautre, de maïs, d'orge malté, de pomme de terre, de carotte, de panais, de patate douce, de topinambour, de poire de terre, de teff, de sorgho, de quinoa, de sarrasin, de seigle, de millet, d'orge, de yakon, de pois, de pois chiche, d'amaranthe, de blé dur, de tritordeum, de tournesol, et leurs mélanges.

10. Hydrolysat obtenu par le procédé selon l'une quelconque des revendications 1 à 9.

11. Hydrolysat selon la revendication 10, lequel est un hydrolysat de tubercule comestible et/ou de graines d'une ou plusieurs plantes herbacées, de préférence ledit hydrolysat de tubercule produit est un hydrolysat de solanacées, d'apiacées, de convolvulaceae, d'astéracées, et leurs mélanges et/ou ledit hydrolysat de graines d'une ou plusieurs plantes herbacées est un hydrolysat de céréales, de pseudo-céréales, de poaceae, d'amaranthaceae, de polygonaceae, de fabaceae, et leurs mélanges.

12. Hydrolysat selon la revendication 11, dans lequel ledit hydrolysat est un hydrolysat de riz, d'avoine, de blé, de manioc, d'épeautre, de maïs, d'orge malté, de pomme de terre, de carotte, de panais, de patate douce, de topinambour, de poire de terre, de teff, de sorgho, de quinoa, de sarrasin, de seigle, de millet, d'orge, de yakon, de pois, de pois chiche, d'amaranthe, de blé dur, de tritordeum, de tournesol, et leurs mélanges.

13. Utilisation de l'hydrolysat selon l'une quelconque des revendications 10 à 12 pour la préparation d'un sirop et/ou d'un sirop déshydraté et/ou d'une base pour produits végétaux.

14. Utilisation selon la revendication 13, dans lequel ledit sirop et/ou ledit sirop déshydraté et/ou ladite base pour produits végétaux, est utilisé dans la préparation d'un produit alimentaire choisi dans le groupe comprenant les boissons (notamment, soda, laitière, végétale), et/ou le jus de fruit et/ou le jus de fruit concentré, les desserts et yaourts (laitiers et végétaux), les biscuits, barres céréalières, en-cas, les céréales pour petit-déjeuner, les biscottes, crackers, les confiseries, les pains, pâtisseries, cakes, gaufres, les crèmes glacées, les sauces, les aliments pour bébé, les produits diététiques/sportifs, les préparations fruitées (notamment, confitures et compotes), et/ou dans la préparation d'un produit cosmétique.
